# FASCICULE DE BREVET EUROPEEN

(11) **EP 3 797 511 B1**
(45) Date de publication et mention de la délivrance du brevet: **22.03.2023**
(21) Numéro de dépôt: 19726053.2
(22) Date de dépôt: 15.04.2019
(51) Int. Cl.: B25J 21/02, H04N 5/225, G06K 9/18, B65B 3/00, B65B 57/00

(54) **DISPOSITIF ET PROCÉDÉ DE RECONNAISSANCE D'UNE ÉTIQUETTE DÉPOSÉE SUR UN OBJET EN VUE DE L'ÉLABORATION SÉCURISÉE DE PRÉPARATIONS MÉDICAMENTEUSES**
VORRICHTUNG UND VERFAHREN ZUR ERKENNUNG EINES AUF EINEM OBJEKT ABGESCHIEDENEN ETIKETTS ZUR SICHEREN HERSTELLUNG VON ARZNEIMITTELZUBEREITUNGEN
DEVICE AND METHOD FOR RECOGNISING A LABEL DEPOSITED ON AN OBJECT IN ORDER TO SECURELY PRODUCE DRUG PREPARATIONS

(30) Priorité: 22.05.2018 FR 1854243
(43) Date de publication de la demande: 31.03.2021
(73) Titulaire: Eurekam, 17140 Lagord (FR)
(72) Inventeur: LE FRANC, Benoît, 17137 NIEUL-SUR-MER (FR); CONAN, Olivier, 17440 AYTRE (FR)
(74) Mandataire: IP Trust
(86) Numéro de dépôt international: PCT/FR2019/050885
(87) Numéro de publication internationale: WO 2019/224438

(56) Documents cités:
- WO-A1-99/10027
- B. C. O'NEAL ET AL: "Telepharmacy and bar-code technology in an i.v. chemotherapy admixture area", AMERICAN JOURNAL OF HEALTH-SYSTEM PHARMACY., vol. 66, no. 13, 17 juin 2009 (2009-06-17) , pages 1211-1217, XP055541917, US ISSN: 1079-2082, DOI: 10.2146/ajhp080388

## Description

### Domaine technique

L'invention se rapporte à un procédé d'élaboration sécurisée, à savoir contrôlée et assistée, de préparations médicamenteuses ainsi qu'à un système d'imagerie apte à mettre en oeuvre un tel procédé.

Le domaine d'application de l'invention concerne la fabrication sécurisée de préparations médicamenteuses permettant un contrôle libératoire de ces préparations. Ce contrôle libératoire est nécessaire, car les préparations médicamenteuses se déroulent selon des protocoles complexes, à partir de mélanges réactionnels entre composants, chaque composant ayant une concentration adaptée à un traitement personnalisé pour un patient donné.

Toute erreur sur la nature d'un composant ou sur sa quantité peut entraîner des conséquences graves sur le patient auquel la préparation est administrée, en particulier lorsqu'une substance active toxique intervient, telles que les préparations de cytotoxiques qui peuvent être utilisées dans les traitements anticancéreux.

Dans la pratique, les opérateurs peuvent travailler plusieurs heures de suite, par exemple dans les unités de préparation des hôpitaux, ce qui augmente sensiblement le risque d'erreur sur la composition ou sur les quantités des composants. La sécurisation des préparations résulte habituellement d'un double contrôle visuel : vérification des étapes clés de chaque préparation et report écrit sur un support de traçabilité approprié, généralement appelé « fiche de fabrication ».

Afin de garantir la qualité des préparations médicamenteuses, l'AFSSAPS (Agence Française de Sécurité Sanitaire des Produits de Santé) a édité les « Bonnes Pratiques de Préparation » qui constituent un texte de référence destiné aux pharmaciens. Les « Bonnes Pratiques de Préparation » précisent les trois obligations auxquelles doit obéir la mise en oeuvre des matières premières utilisées dans les préparations médicamenteuses :
- la méthode de mesure des quantités de matières premières est choisie en fonction de leur nature et de la quantité à mesurer;
- la mesure du volume ou la pesée des quantités de matières premières fait l'objet d'enregistrements ;
- les matières premières sont identifiables de manière permanente au cours des opérations précitées.

Ainsi, lors de la préparation, la nature de chaque matière première utilisée ainsi que sa masse ou son volume sont vérifiés indépendamment soit par un moyen d'enregistrement automatique, soit par une seconde personne qualifiée au sens du Code de la Santé Publique, et la vérification est notée dans le dossier de lot de la préparation. Pour répondre aux « Bonnes Pratiques de Préparation » et pour la sécurité des patients, il est donc recommandé d'effectuer un double contrôle de la nature et des quantités des compositions utilisées dans chaque préparation.

### État de la technique antérieure

Le document WO99/10027 A1 divulgue un dispositif de reconnaissance de données d'informations présentes sur une surface d'un objet tel qu'un flacon, une seringue ou une ampoule, en vue de l'élaboration sécurisée de préparations médicamenteuses. U

De la publication WO 2014/202859 A1 de la demanderesse, on connaît un procédé et dispositif de prise de vue pour l'élaboration sécurisée de préparations médicamenteuses et un support de positionnement d'objets associés.

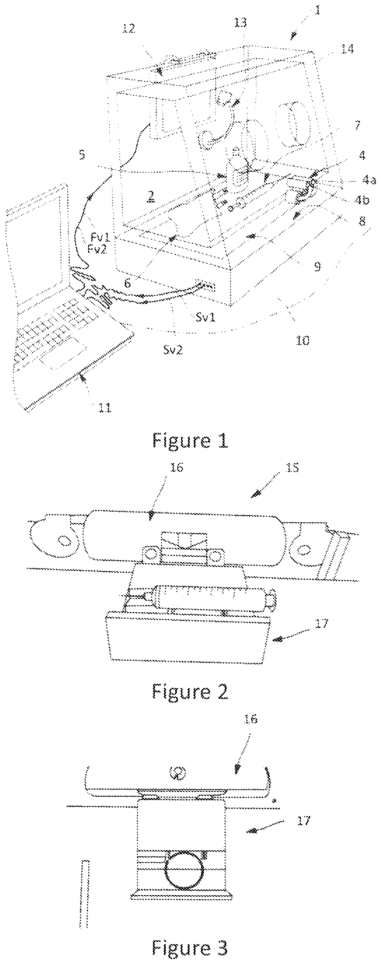

En référence à la vue en perspective de la figure 1, qui reprend la figure 1 de la publication précitée, il est maintenant rappelé le contexte de suivi de la préparation médicamenteuse développé par la demanderesse.

Une hotte portative 1 intègre un site 2 de préparation de médicaments. Ce site 2 est équipé d'un système d'imagerie 3 (non représenté) permettant d'élaborer de telles préparations de manière sécurisée. Ce système d'imagerie 3 comporte une caméra 4 dite de traitement. La focale de cette caméra 4 est réglée pour réaliser une mise au point sur des contenants et des outils d'administration de médicaments, ici un flacon 5, une poche de perfusion 6 contenant une solution saline, et une seringue 7. Dans l'exemple, le flacon 5 contient du cisplatine à diluer dans la poche de perfusion 6. Pour ce faire, un prélèvement est effectué par l'opérateur dans le flacon 5 de cisplatine à l'aide de la seringue 7 puis injecté dans la poche de perfusion 6. L'opération est reproduite jusqu'à l'obtention de la dilution voulue du principe actif dans la poche pour préparer, dans l'exemple, un composant de polychimiothérapie.

Avantageusement, un fond 8 de la hotte 1 possède une conformation de structure surfacique adaptée aux contours des objets, favorisant une dépose stabilisée et reproductible des flacons et seringues, et intègre un dispositif de rétroéclairage 9 permettant de mieux lire les graduations de la seringue 7.

La caméra de traitement 4 est positionnée au niveau du fond de hotte 8 légèrement au-dessus d'un plan de travail sur lequel est posée la hotte portative 1, c'est-à-dire sensiblement, dans l'exemple, au niveau ventral d'un l'opérateur (non représenté).

La caméra de traitement 4 est connectée à une unité de gestion numérique, un ordinateur portable 11 dans l'exemple. L'ordinateur 11 comporte principalement un processeur et des mémoires (non représentés) qui traitent des signaux vidéo Sv1 provenant de la caméra de traitement 4 pour fournir des images à un écran d'affichage 12 et les enregistrer.

L'écran d'affichage 12 permet alors de visualiser des informations correspondant à la préparation en cours à partir d'un flux vidéo Fv1 fourni par la caméra de traitement 4.

Une autre caméra 13 d'enregistrement et de visualisation, dite caméra de scène, possède une focale réglée pour une détection globale du site 2. L'objectif de la caméra de scène 13 est avantageusement positionné en un niveau supérieur 14 de la hotte 1 de sorte à permettre une vision globale du site 2 par transmission d'un flux vidéo Fv2 à l'écran d'affichage 12.

Les caméras de traitement 4 et de scène 13 fournissent des signaux vidéo S_{V1}, S_{V2} synchronisés par le processeur de l'ordinateur 11 afin de constituer une interface graphique dynamique pour un contrôle en temps réel et *a posteriori.*

La caméra de traitement 4 est avantageusement composée de deux caméras accolées 4a, 4 b, de focales réglées sur la détection d'objets - flacons et seringues en général - dans des gammes de dimensions complémentaires, inférieure à 3 cm et compris entre 3 et 307 cm dans l'exemple.

Les flux vidéo des caméras de traitement 4a et 4b sont analysés par un outil de reconnaissance de forme et de caractère, intégré au processeur de l'ordinateur 11.

L'analyse permet une identification automatique des objets utilisés, flacon 5 et seringue 7, par un traitement des premiers flux vidéo à l'aide de l'outil de reconnaissance. La détection des volumes de produit contenus dans les seringues et les flacons permet un contrôle non destructeur du principe actif mis en oeuvre dans la préparation.

Ainsi, toutes les données figurant sur du flacon 5, en particulier la concentration du principe actif, sont identifiées par ledit outil de reconnaissance. Ces données sont mémorisées dans l'ordinateur 11. Au cours de la préparation, des données d'analyse des contenus du flacon et de la seringue sont affichées sur l'écran 12 et enregistrées en vue de la validation de chaque étape de la préparation : quantité de liquide dans la seringue 7 et niveau de liquide dans le flacon 5.

Le processeur de l'ordinateur 11 recherche dans une mémoire, où est stocké un ensemble de prescriptions, la prescription correspondant à la préparation en cours. Pour ce faire, les prescriptions mémorisées sont indexées par étapes à l'aide d'un outil d'indexation numérique. Les étapes clés - qui sont les étapes spécifiques propres à chaque prescription.

Le processeur compare les données mémorisées de la préparation en cours fournies par les premières images enregistrées - nom des composants, quantités versées dans les seringues, etc. - et les étapes des prescriptions mémorisées. Dès qu'une étape clé de cette prescription est reconnue, la prescription correspondant à la préparation en cours est alors identifiée et affichée par ses étapes. Les informations sont sélectionnées en fonction de la prescription détectée parmi une liste de flacons disponibles et une liste de flacons utilisés mémorisées dans l'ordinateur en fonction de données fournies par le centre de gestion du laboratoire.

Un affichage d'alerte est intégré, ainsi que la possibilité d'interaction manuelle pour prélever ou injecter un volume déterminé, ou encore ajouter un flacon.

De plus, l'état d'avancement de la préparation est affiché en fonction des données du protocole mémorisé correspondant à la prescription identifiée : des informations concernant la gestion du volume contenu dans le flacon 5 et l'évolution des volumes du composant injectés/manquants/prescrits dans la seringue 7 sont affichées. Ces informations de gestion sont initiées par une incrémentation des volumes injectés et en conséquence du volume restant, du nombre d'injections, du nombre de flacons et des reliquats. Cette incrémentation est automatisée à partir de l'interface graphique des flux vidéo synchronisés des caméras 4 et 13. Une telle gestion permet de baisser sensiblement les erreurs dues aux manipulations.

L'affichage d'alerte est activé en temps réel dès que le flacon et/ou les volumes de composant détectés avant injection dans une étape ne sont pas conformes au protocole mémorisé de la prescription identifiée. Les étapes détectées en erreur sont identifiées par comparaison avec les étapes de la prescription. La non-conformité d'une étape clé déclenche une recherche d'erreur dans l'identification de la prescription qui a été sélectionnée.

L'affichage fournit alors des directives et l'alerte n'est supprimée et la préparation ne se poursuit que si la conformité entre les étapes de la préparation et celles constituant le protocole de ladite prescription est validée. L'avancement de la préparation est alors réactualisé en temps réel jusqu'à la validation finale. La préparation est ainsi sécurisée par une réactivité quasi instantanée et la reproductibilité des préparations est optimisée.

La validation des étapes détectées en erreur, en particulier des étapes clés de la préparation, permet de poursuivre la préparation jusqu'à son terme.

Ainsi, la préparation peut se faire sous hotte fixe ou tout environnement adapté pour installer le système d'imagerie.

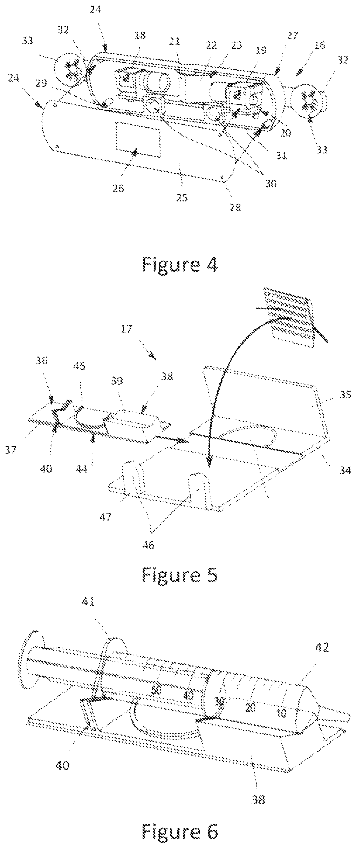

En référence aux figures 2 à 7, qui reprennent les figures 4 à 9 de la publication précitée, il est rappelé un exemple de système de prise de vue mettant en oeuvre le procédé d'élaboration sécurisée de préparations médicamenteuses est décrit.

Ce système est disposé de part et d'autre d'une baie transparente 15 d'une enceinte d'élaboration sécurisée d'une préparation médicamenteuse, et comprend un dispositif de prise de vue 16 disposé à l'extérieur de l'enceinte contre la paroi transparente 15, et un support 17 de positionnement d'objets disposé à l'intérieur de l'enceinte en vis-à-vis du dispositif de prise de vue.

Plus précisément, comme mieux visible sur la figure 4, le dispositif de prise de vue 16 comprend deux caméras de traitement 18, 19 disposées aux deux extrémités d'une embase 20 allongée et rectangulaire, un élément réfléchissant 21, 22 pour chacun des appareils de prise de vue 18, 19, disposés à mi-longueur de l'embase 20.

Les deux éléments réfléchissants 21, 22 sont orientés le long de l'axe défini par les deux caméras, de façon à réfléchir en direction de la caméra correspondante, des images du support 17 de positionnement d'objets.

Plus précisément, ces deux éléments réfléchissants sont formés par les deux faces rectangulaires adjacentes d'un prisme droit à base triangulaire 23, le prisme étant monté sur l'embase par l'une de ses bases triangulaires.

Les deux caméras 18, 19 présentent des objectifs de focales différentes permettant de faire le point sur des objets de tailles différentes se trouvant sur le support de seringues et flacons. Par exemple, la caméra de droite peut être consacrée à la mise au point pour des flacons et seringues de petite taille avec un objectif de 12 mm, celle de gauche pouvant être consacrée à la mise au point pour des flacons et seringues de grande taille avec un objectif de 8 mm.

Afin de doter le dispositif de prise de vue de faibles dimensions, les deux caméras présenteront elles-mêmes de faibles dimensions, par exemple 47x 29 x 29 mm (L x l x h). Un exemple de caméras convenant à cette utilisation est de la marque Imaging source^{®}, référence DFK23F445.

L'embase allongée 20 est fixée à l'intérieur d'un coffret 24 comprenant une face avant 25 plane et sensiblement rectangulaire, dont les deux côtés courts sont légèrement arrondis, et munie d'une fenêtre centrale de visibilité 26 sensiblement rectangulaire, et une coque arrière allongée 27 à l'intérieur de laquelle l'embase 20 est solidairement fixée, par son bord arrière longitudinal, le prisme 23 se retrouvant dans l'ouverture de la fenêtre 26 lorsque la face avant 25 du coffret referme la coque 27, afin que les images du support de seringues et flacons parviennent via ce prisme, aux deux caméras 18, 19.

La face avant 25 peut être fixée à la coque 27 au moyen de vis traversant des trous 28 prévus à cet effet aux quatre coins de la face avant 25 et venant se visser dans un fourreau correspondant 29 prévu à cet effet sur la paroi interne de la coque arrière 27.

La coque arrière 27 comprend en outre deux pattes 30 s'étendant verticalement depuis son bord latéral inférieur 31, ces pattes étant pourvues d'un évidemment central circulaire d'accueil d'un moyen magnétique de forme complémentaire (pastille métallique ou aimantée, non représentée), destiné à retenir contre la baie vitrée 15 et la face avant 25 du coffret, le support pour seringues et flacons 17 qui est pourvu de moyens aimantés complémentaires.

La coque 27 comprend en outre deux lames latérales rigides 32 fixées sur la face externe de sa paroi arrière et dépassant de part et d'autre de ses extrémités, ces deux lames 32 portant deux ventouses 33 dirigées vers l'avant afin d'être fixées sur la baie vitrée de l'enceinte.

D'autre part, conformément à la figure 5, le support 17 pour seringues et flacons comprend une base 34 plane de support, un écran incliné 35 dressé en arrière de la base destiné à former un fond blanc, et un porte-seringue 36 monté à translation vis-à-vis de la base 34 entre une position désengagée indépendante de la base 34 (illustrée sur la figure 5) et une position engagée solidaire de la base 34 (illustrée sur la figure 2).

Plus précisément, le porte seringue 36 comprend une plaque rectangulaire 37, à une extrémité de laquelle fait saillie un pavé 38 de forme sensiblement rectangulaire, mais dont la paroi supérieure est en creux longitudinal 39 (la paroi supérieure est composée de pans inclinés convergeant l'un vers l'autre et se rejoignant par un bord commun, définissant une section transversale en forme de « M ») afin de servir de support pour le corps d'une seringue, et de retenir latéralement par les pans inclinés, ce corps de seringue.

À l'autre extrémité, la plaque 37 du porte seringue 36 comprend un chevalet 40 de retenue des ailes 41 d'une seringue 42. Le chevalet 40 est formé par deux parois parallèles, séparées l'une de l'autre d'une distance suffisante pour accueillir les ailes 41 de la seringue 42 (voir figure 6), et présentent, à l'image du pavé 38, un contour en forme de « M » afin de supporter et retenir latéralement la partie de seringue à proximité des ailes 41.

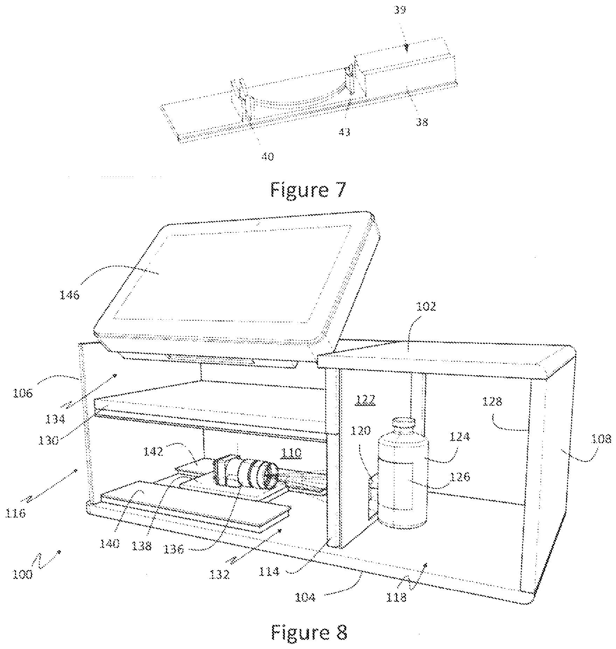

Conformément à la figure 7, il est prévu, pour le support de seringues de petites tailles, un chevalet supplémentaire 43 entre le pavé 38 et le chevalet principal 40, en étant plus proche du pavé 38, et se présentant sous la forme d'une unique paroi 43 à contour en forme de « M », dont le sillon se retrouve au même niveau que celui du pavé 38.

La plaque 37 du porte-seringue 36 comprend, conformément à la figure 5, deux bords latéraux amincis 44 susceptibles de coulisser dans des gorges formées sur les bords latéraux d'un creux d'accueil de la plaque ménagé dans la base 34, afin de pouvoir engager par coulissement le support de seringues dans la base jusqu'à une position engagée (figure 2).

Dans cette position engagée, le support de seringues 36 et la base 34 définissent ensemble un relief 45 d'accueil pour le cul d'un flacon.

En effet, les surfaces supérieures de la base 34 et de la plaque 37 du porte seringue 36 sont coplanaires lorsque le porte seringues occupe sa position engagée et comprennent des creux complémentaires 45 d'accueil d'un flacon en forme de demi disque, et qui forment lorsque le porte seringue occupe sa position engagée, un creux d'accueil circulaire du cul d'un flacon, ce creux se retrouvant disposé entre le chevalet de retenue 40 et le pavé d'appui 38. Bien entendu, un autre relief, tel qu'une nervure circulaire, peut remplir la même fonction.

La base 34 porte les moyens magnétiques complémentaires 46 de ceux prévus sur les pattes 30 du dispositif de prise de vue. Plus précisément, ces moyens magnétiques (pastille aimantée ou métallique, suivant celle utilisée pour les moyens magnétiques du dispositif de prise de vue) sont logés à l'intérieur de deux ergots 47 faisant saillie verticalement du bord de la base 34 opposé à l'écran 35, et présentant une paroi avant plane pouvant se plaquer contre la face interne de la baie vitrée, au niveau des pattes correspondantes 30 du dispositif de prise de vue.

Le système tel que décrit ci-dessus permet la mise en oeuvre du procédé d'élaboration sécurisé de préparations médicamenteuse, même lorsque des seringues et des flacons de différentes tailles sont utilisés, grâce à la pluralité d'appareils de prise de vue à objectifs différents utilisés et aux éléments réfléchissants permettant à ces appareils de recueillir des images de la même zone : le support de seringues et de flacons.

Cet objectif est en outre atteint avec un encombrement faible du fait de l'utilisation d'un prisme central pour les deux appareils de prise de vue.

Toutefois, la demanderesse a continué d'innover.

La demanderesse a noté que son système n'était pas adapté pour être utilisé avec différentes inclinaisons de baies transparentes par rapport au plan de travail. En effet, entre deux sites prédéterminés sur lesquels la préparation médicamenteuse doit être réalisée, les inclinaisons des baies peuvent être différentes, obligeant la demanderesse à adapter son système à chacune des inclinaisons de baies.

La demanderesse a en outre noté que le positionnement du système décrit dans la publication précitée pouvait être imprécis, notamment du fait de l'utilisation de ventouse. En effet, à l'installation, une calibration est nécessaire. Le nettoyage de la baie 15 peut imposer le retrait du dispositif de prise de vue et donc de ses ventouses. Il est ensuite alors difficile de le repositionner à l'identique, ce qui peut entraîner des problèmes de prises de vue.

La demanderesse a aussi noté que les opérateurs sont sujets à des troubles musculosquelettiques (TMS) et que la présence de l'écran implique une gestuelle entraînant des tendinites de la coiffe des rotateurs de l'épaule de l'opérateur lorsque l'opérateur met en oeuvre le produit.

Enfin, la demanderesse a noté que le contrôle de l'environnement lumineux peut être amélioré.

### Exposé de l'invention

Un but de l'invention est notamment de remédier à tout ou partie des inconvénients précités.

A cet effet, il est proposé un dispositif de reconnaissance de données d'informations présentes sur une surface d'un objet tel qu'un flacon, une seringue ou une ampoule, en vue de l'élaboration sécurisée de préparations médicamenteuses, comportant :
- une enceinte, dite de positionnement, agencée pour recevoir l'objet et présentant une face, dite de référence, d'une couleur prédéterminée (par exemple blanche ou autre),
- une enceinte, dite de capture, agencée pour recevoir un dispositif de capture d'image, le dispositif de capture d'image présentant un axe optique orienté en direction de la face de référence.

L'enceinte de positionnement et l'enceinte de capture présentent une paroi commune, ladite paroi commune présentant une surface cible, disposée de sorte que l'axe optique dudit dispositif de capture coupe ladite surface cible, ladite surface cible étant réalisée sous forme d'orifice débouchant dans l'enceinte de positionnement et l'enceinte de capture, ou dans un matériau rigide et transparent.

Le dispositif selon l'invention comporte en outre une unité de calcul, configurée pour réaliser une reconnaissance optique de caractère à partir d'une image capturée par le dispositif de capture d'image, ladite unité de calcul étant en outre configurée pour générer des données de sortie à destination d'un périphérique de sortie.

Aussi, il devient alors possible d'éviter des troubles musculosquelettiques (TMS) puisque l'objet est facilement insérable dans l'enceinte de positionnement, contre la paroi formée par la surface cible.

Les problèmes liés au positionnement du système de l'état de l'art sont résolus, ainsi que ceux liés à l'inclinaison des baies vitrées.

Enfin, les données de sorties permettent d'alerter le préparateur lorsque des données d'informations ne correspondant pas à ses attentes sont reconnues, ou de le rassurer lorsque les données d'informations reconnues sont conformes à ses attentes.

Les données d'informations peuvent être portées par une étiquette déposée sur la surface de l'objet.

Alternativement, ou en complément, des données d'informations peuvent être directement portées sur la surface de l'objet, par exemple au moyen d'imprimante à encre, de technique de sérigraphie, ou encore de gravure.

Les données d'informations peuvent être représentées sous forme de chiffres, par exemple de chiffre formant un nombre indiquant un volume, comme ce peut être le cas dans le cas de graduation de seringue.

Les données d'informations peuvent être représentées sous forme de caractères et/ou de chiffres, par exemple pour indiquer une substance et une concentration.

Les données d'informations peuvent être encodées sous forme de codes-barres en deux dimensions, par exemple sous forme de codes connus sous le nom de QR code ou bien encore de data matrix.

L'unité de calcul peut être directement insérée dans le dispositif ou bien être déportée.

Le périphérique de sortie peut être un haut-parleur. Par exemple un haut-parleur d'un écran ordinateur comportant l'unité de calcul ou encore un haut-parleur déporté. Les données de sorties sont alors des informations vocales, par exemple enregistrées au sein d'une base de données.

Le dispositif selon l'invention peut en outre comporter un dispositif d'affichage, configuré pour afficher une image générée par l'unité de calcul.

L'image générée par l'unité de calcul peut être réalisée à partir d'une image capturée par le dispositif de capture d'image et/ou à partir des données de sorties.

Avantageusement, le dispositif selon l'invention peut en outre comporter un dispositif d'éclairage d'une zone disposée dans l'enceinte de capture, sur l'axe optique, entre le dispositif de capture et la paroi commune.

Selon une possibilité, le dispositif d'éclairage peut être un panneau lumineux.

Selon un mode de réalisation, le dispositif selon l'invention peut comporter une batterie électrique interne. Le dispositif est alors autonome en énergie et aisément déplaçable.

A titre d'exemple, le dispositif selon l'invention peut être déposé sur une structure roulante, comportant par exemple un châssis et des roulettes.

Selon un autre aspect de l'invention, il est proposé un procédé de reconnaissance de données d'informations présentes sur une surface d'un objet tel qu'un flacon, une ampoule ou une seringue, en vue de l'élaboration sécurisée de préparations médicamenteuses, comportant :
- un positionnement dans une enceinte, dite de positionnement, de l'objet, l'enceinte de positionnement présentant une face, dite de référence, d'une couleur prédéterminée,
- une capture dans une enceinte, dite de capture, par un dispositif de capture d'image, le dispositif de capture d'image présentant un axe optique orienté en direction de la face de référence.

Selon le deuxième aspect de l'invention, l'enceinte de positionnement et l'enceinte de capture présentent une paroi commune, ladite paroi commune présentant une surface cible, disposée de sorte que l'axe optique dudit dispositif de capture coupe ladite surface, ladite surface cible étant réalisée sous forme d'orifice débouchant dans l'enceinte de positionnement et l'enceinte de capture, ou dans un matériau rigide et transparent.

Le procédé selon le deuxième aspect de l'invention comportant en outre :
- une reconnaissance optique de caractère par une unité de calcul à partir d'une image capturée par le dispositif de capture d'image
- une génération de données de sortie par l'unité de calcul, à destination d'un périphérique de sortie

### Présentation des figures

D'autres données, caractéristiques et avantages de la présente invention apparaîtront à la lecture de la description de mises en oeuvre et de modes de réalisation nullement limitatifs, au regard de dessins annexés sur lesquels :
- la figure 1 est une vue en perspective d'un site de préparations médicamenteuses équipé d'un exemple de système d'imagerie sécurisé de telles préparations ;
- la figure 2 est une vue en perspective de face d'un système de prise de vue de seringues et de flacons de tailles différentes, se positionnant sur la baie transparente d'une enceinte de préparation, un support pour seringues et flacons, interne à l'enceinte, et un dispositif de prise de vue externe à l'enceinte,
- la figure 3 est une vue en perspective de dessus du système de la figure 2,
- la figure 4 est une vue éclatée du dispositif de la figure 2,
- la figure 5 est une vue éclatée du support pour seringues et flacons de la figure 2, représentant un porte-seringue en position désengagée vis-à-vis d'une base du support,
- la figure 6 est une vue en perspective du côté droit du support soutenant une seringue,
- la figure 7 est une vue en perspective du côté gauche du support sans seringue,
- la figure 8 est une vue en perspective de l'avant, au-dessus et depuis la droite d'un mode de réalisation d'un dispositif de prise de vue selon l'invention,
- la figure 9 est une vue en perspective de l'avant, en dessous et depuis la gauche, du dispositif de prise de vue illustré sur la figure 8,
- la figure 10 est une vue en perspective depuis la gauche et au-dessus du dispositif de prise de vue illustré sur la figure 9.

### Description des modes de réalisation

Les modes de réalisation décrits ci-après n'étant nullement limitatifs, on pourra notamment considérer des variantes de l'invention ne comprenant qu'une sélection de caractéristiques décrites, par la suite isolées des autres caractéristiques décrites, si cette sélection de caractéristiques est suffisante pour conférer un avantage technique ou pour différencier l'invention par rapport à l'état de la technique antérieure. Cette sélection comprend au moins une caractéristique, de préférence fonctionnelle sans détails structurels, ou avec seulement une partie des détails structurels si cette partie uniquement est suffisante pour conférer un avantage technique ou pour différencier l'invention par rapport à l'état de la technique antérieure.

En référence à la figure 8, il est maintenant décrit un dispositif de reconnaissance d'étiquette 100 selon l'invention.

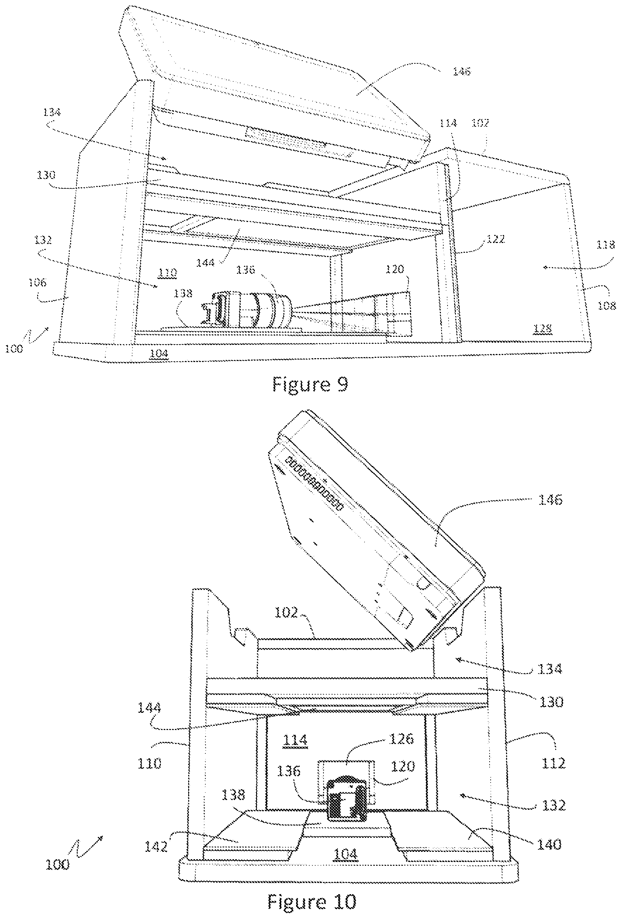

Le dispositif 100 a une forme générale de pavé, doté d'une face supérieure 102, d'une face inférieure 104, de deux faces latérales 106 et 108, respectivement gauche et droite, d'une face arrière 110 et d'une face avant 112 (voir figures 9 et 10).

Le dispositif de prise de vue 100 est divisé, au moyen d'une paroi de séparation 114 verticale opaque, en deux enceintes, respectivement 116 de capture et 118 de positionnement.

La face arrière 110 et la face avant 112 s'étendent depuis la face latérale gauche 106 jusqu'à la paroi de séparation 114, laissant le compartiment 118 accessible par l'arrière et l'avant.

La face supérieure 102 s'étend depuis la paroi de séparation jusqu'à la face latérale droite 108.

Une surface cible 120, sous forme d'orifice 120, de forme rectangulaire selon l'exemple illustré, est ménagé dans la paroi de séparation 114.

Toujours dans l'exemple illustré, une plaque de verre 122 transparente de forme sensiblement identique à la paroi de séparation 114 est disposée contre cette dernière, du côté du compartiment 118.

Selon un autre exemple de réalisation, la paroi de séparation 114 et plaque de verre 122 pourraient être réunies en une seule pièce, par exemple de verre sablé, sauf au niveau de la partie d'orifice, qui serait alors transparente.

La plaque de verre 122 constitue, du côté du compartiment 118, une surface d'appui pour présenter un flacon 124, muni d'une étiquette 126.

Du côté intérieur au compartiment 118, la paroi 108 est dotée d'une face de référence 128, de couleur blanche.

Le compartiment 116 est divisé, au moyen d'une plaque de séparation 130 horizontale, s'étendant horizontalement entre la face latérale gauche 106 et la paroi de séparation 114 en deux logements, 132 et 134, respectivement bas et haut.

Une caméra 136, en tant que dispositif de capture d'image, est disposée dans le logement 132 et son axe optique est dirigé vers le fond blanc 128 et traverse l'orifice 120.

Plus précisément, dans l'exemple représenté, l'orifice 120 est intégralement ménagé dans la paroi de séparation 114, sans déboucher dans le logement 118.

Toujours sur l'exemple représenté, l'axe optique de la caméra 136 est perpendiculaire au plan de l'orifice 120 et centré au milieu de l'orifice 120.

Dans le mode de réalisation représenté, la caméra 136 est fixée à un support de caméra 138, lui-même monté à coulissement selon un axe parallèle à l'axe optique, sur deux glissières, 140 et 142, respectivement avant et arrière.

Dans une variante de ce premier mode de réalisation d'un dispositif selon l'invention, uniquement décrite pour sa différence avec ce premier mode de réalisation, la caméra pourrait être solidaire du dispositif de prise de vue 100, par exemple directement fixée sur la face inférieure 104.

Comme mieux visible en figure 2 et 3, une plaque d'illumination 144 de type LED, en tant que dispositif d'éclairage, est montée sur la plaque de séparation 130, du côté du logement 132.

Aussi, l'environnement de prise de vue, tant du côté du compartiment 118 que du logement 132, est maîtrisé, tant du point de vue de la lumière et du fonds, mais aussi de la distance de l'objet à la caméra.

La face supérieure 102 permet aussi de mieux maîtriser l'environnement, empêchant par exemple des rayons lumineux émis par un néon de venir perturber l'environnement du dispositif de prise de vue.

Le logement 134 est agencé pour recevoir, de manière escamotable, un ordinateur-écran 146.

L'ordinateur-écran est en outre muni de haut-parleurs.

Lorsqu'un objet124 présentant une étiquette 126 est placé devant l'orifice 120, l'objet est détecté par la caméra 136.

La caméra 136 réalise alors plusieurs captures d'images, qui sont envoyées au microprocesseur de l'ordinateur-écran 146.

Le microprocesseur est configuré pour réaliser une reconnaissance de type OCR des caractères inscrit sur l'étiquette 126.

Lorsque cette reconnaissance est réussie, le microprocesseur est configuré pour jouer un son, via les haut-parleurs, associé à l'étiquette 126.

Le microprocesseur peut en outre être configuré pour faire afficher les captures d'images par l'écran.

Le microprocesseur peut en outre être configuré pour horodater le cas échéant l'image reconnue et par exemple inscrire l'heure, le jour, le nom de l'établissement et un numéro de salle, assurant ainsi une traçabilité.

L'ordinateur-écran peut en outre être doté d'une webcam.

L'ordinateur-écran peut en outre être doté d'une connectique réseau, de type filaire ou non.

L'ordinateur-écran peut en outre être doté d'une sortie USB.

Aussi, l'opérateur peut avoir une confirmation orale, et/ou visuelle au moyen de l'écran, du contenu de l'objet, via la lecture de l'étiquette.

L'objet pourrait être une ampoule, ou encore une seringue, ou une simple étiquette.

Bien entendu, le haut-parleur, l'écran et même l'ordinateur pourraient être distants. En ce cas, le logement 134 pourrait être doté d'une surface de préparation pour poser un plateau de préparation.

Le dispositif de prise de vue 100 peut être alimenté en énergie, ou être autonome en énergie.

Le dispositif de prise de vue 100 peut être doté d'une structure roulante, par exemple de roulettes.

Bien sûr, l'invention n'est pas limitée aux exemples qui viennent d'être décrits et de nombreux aménagements peuvent être apportés à ces exemples sans sortir du cadre de l'invention. De plus, les différentes caractéristiques, formes, variantes et modes de réalisation de l'invention peuvent être associés les uns avec les autres selon diverses combinaisons dans la mesure où ils ne sont pas incompatibles ou exclusifs les uns des autres.

## Revendications

1. Dispositif (100) de reconnaissance de données d'informations (126) présentes sur une surface d'un objet (124) tel qu'un flacon, une seringue ou une ampoule, en vue de l'élaboration sécurisée de préparations médicamenteuses, comportant :
- une enceinte (118), dite de positionnement, agencée pour recevoir l'objet et présentant une face (128), dite de référence, d'une couleur prédéterminée,
- une enceinte (132), dite de capture, dans laquelle est disposé un dispositif de capture d'image (136), le dispositif de capture d'image présentant un axe optique orienté en direction de la face de référence,
dans lequel l'enceinte de positionnement et l'enceinte de capture présentent une paroi commune (114),
ladite paroi commune présentant une surface cible (120), disposée de sorte que l'axe optique dudit dispositif de capture coupe ladite surface cible,
ladite surface cible étant réalisée sous forme d'orifice débouchant dans l'enceinte de positionnement et l'enceinte de capture, ou dans un matériau rigide et transparent,
- une unité de calcul (146), configurée pour réaliser une reconnaissance optique de caractère à partir d'une image capturée par le dispositif de capture d'image,
ladite unité de calcul étant en outre configurée pour générer des données de sortie à destination d'un périphérique de sortie.

2. Dispositif selon la revendication précédente, dans lequel les données d'informations sont portées par une étiquette déposée sur la surface de l'objet.

3. Dispositif selon la revendication 1, dans lequel les données d'informations sont portées directement sur la surface de l'objet.

4. Dispositif selon l'une quelconque des revendications précédentes, dans lequel les données d'informations sont représentées sous forme de caractères et/ou de chiffres.

5. Dispositif selon l'une quelconque des revendications précédentes, dans lequel les données d'informations sont encodées sous forme de codes-barres en deux dimensions.

6. Dispositif selon l'une quelconque des revendications précédentes, dans lequel le périphérique de sortie est un haut-parleur.

7. Dispositif selon l'une quelconque des revendications précédentes, comportant en outre un dispositif d'affichage, configuré pour afficher une image générée par l'unité de calcul.

8. Dispositif selon la revendication précédente, dans lequel l'image générée par l'unité de calcul est réalisée à partir d'une image capturée par le dispositif de capture d'image et/ou des données de sorties.

9. Dispositif selon l'une quelconque des revendications précédentes, comportant en outre un dispositif d'éclairage (144) d'une zone disposée dans l'enceinte de capture, sur l'axe optique, entre le dispositif de capture et la paroi commune.

10. Dispositif selon la revendication précédente, dans lequel le dispositif d'éclairage est un panneau lumineux LED.

11. Dispositif selon l'une quelconque des revendications précédentes, comportant en outre une batterie électrique interne.

12. Dispositif selon l'une quelconque des revendications précédentes, doté en outre d'une structure roulante, par exemple de roulettes.

13. Procédé de reconnaissance de données d'informations présentes sur une surface (126) d'un objet (124) tel qu'un flacon, une seringue ou une ampoule, en vue de l'élaboration sécurisée de préparations médicamenteuses, comportant :
- un positionnement dans une enceinte (118), dite de positionnement, de l'objet, l'enceinte de positionnement présentant une face (128), dite de référence, d'une couleur prédéterminée,
- une capture dans une enceinte (132), dite de capture, par un dispositif de capture d'image (136), le dispositif de capture d'image présentant un axe optique orienté en direction de la face de référence,
dans lequel l'enceinte de positionnement et l'enceinte de capture présentent une paroi commune (114),
ladite paroi commune présentant une surface cible (120), disposée de sorte que l'axe optique dudit dispositif de capture coupe ladite surface, ladite surface cible étant réalisée sous forme d'orifice débouchant dans l'enceinte de positionnement et l'enceinte de capture, ou dans un matériau rigide et transparent,
- une reconnaissance optique de caractère par une unité de calcul (146) à partir d'une image capturée par le dispositif de capture d'image,
- une génération de données de sortie par l'unité de calcul, à destination d'un périphérique de sortie.

## Patentansprüche

1. Vorrichtung (100) zum Erkennen von Informationsdaten (126), die auf einer Oberfläche eines Gegenstands (124) wie zum Beispiel einem Fläschchen, einer Spritze oder einer Ampulle vorhanden sind, im Hinblick auf die sichere Herstellung von Arzneimittelzubereitungen, die Folgendes umfasst:
- ein sogenanntes Positionierungsgehäuse (118), das zum Aufnehmen des Gegenstands eingerichtet ist und eine sogenannte Bezugsfläche (128) mit einer zuvor bestimmten Farbe aufweist,
- ein sogenanntes Erfassungsgehäuse (132), in dem eine Bilderfassungsvorrichtung (136) angeordnet ist, wobei die Bilderfassungsvorrichtung eine in Richtung der Bezugsfläche ausgerichtete optische Achse aufweist,
wobei das Positionierungsgehäuse und das Erfassungsgehäuse eine gemeinsame Wand (114) aufweisen,
wobei die gemeinsame Wand eine Zieloberfläche (120) aufweist, die angeordnet ist, so dass die optische Achse der Erfassungsvorrichtung die Zieloberfläche schneidet,
wobei die Zieloberfläche in Form einer Öffnung, die in das Positionierungsgehäuse und das Erfassungsgehäuse mündet, oder aus einem starren und transparenten Material ausgebildet ist,
- eine Recheneinheit (146), die zum Ausbilden einer optischen Zeichenerkennung aus einem durch die Bilderfassungsvorrichtung erfassten Bild konfiguriert ist,
wobei die Recheneinheit ferner zum Erzeugen von Ausgabedaten an eine Ausgabeeinrichtung konfiguriert ist.

2. Vorrichtung nach dem vorhergehenden Anspruch, wobei die Informationsdaten durch ein Etikett getragen werden, das auf der Oberfläche des Gegenstands angebracht ist.

3. Vorrichtung nach Anspruch 1, wobei die Informationsdaten direkt auf der Oberfläche des Gegenstands getragen werden.

4. Vorrichtung nach einem der vorhergehenden Ansprüche, wobei die Informationsdaten in Form von Zeichen und/oder Zahlen dargestellt sind.

5. Vorrichtung nach einem der vorhergehenden Ansprüche, wobei die Informationsdaten in Form von zweidimensionalen Strichcodes codiert sind.

6. Vorrichtung nach einem der vorhergehenden Ansprüche, wobei die Ausgabeeinrichtung ein Lautsprecher ist.

7. Vorrichtung nach einem der vorhergehenden Ansprüche, die ferner eine Anzeigevorrichtung umfasst, die zum Anzeigen eines durch die Recheneinheit erzeugten Bildes konfiguriert ist.

8. Vorrichtung nach dem vorhergehenden Anspruch, wobei das durch die Recheneinheit erzeugte Bild aus einem durch die Bilderfassungsvorrichtung erfassten Bild und/oder Ausgabedaten ausgebildet wird.

9. Vorrichtung nach einem der vorhergehenden Ansprüche, die ferner eine Beleuchtungsvorrichtung (144) für einen Bereich umfasst, der in dem Erfassungsgehäuse auf der optischen Achse zwischen der Erfassungsvorrichtung und der gemeinsamen Wand angeordnet ist.

10. Vorrichtung nach dem vorhergehenden Anspruch, wobei die Beleuchtungsvorrichtung ein LED-Leuchtfeld ist.

11. Vorrichtung nach einem der vorhergehenden Ansprüche, die ferner eine interne elektrische Batterie umfasst.

12. Vorrichtung nach einem der vorangehenden Ansprüche, die ferner mit einer rollenden Struktur, beispielsweise Laufrollen, ausgestattet ist.

13. Verfahren zum Erkennen von Informationsdaten, die auf einer Oberfläche (126) eines Gegenstands (124) wie zum Beispiel einem Fläschchen, einer Spritze oder einer Ampulle vorhanden sind, im Hinblick auf die sichere Herstellung von Arzneimittelzubereitungen, das Folgendes umfasst:
- Positionieren des Gegenstands in einem sogenannten Positionierungsgehäuse (118), wobei das Positionierungsgehäuse eine sogenannte Bezugsfläche (128) mit einer zuvor bestimmten Farbe aufweist,
- Erfassen in einem sogenannten Erfassungsgehäuse (132) durch eine Bilderfassungsvorrichtung (136), wobei die Bilderfassungsvorrichtung eine in Richtung der Bezugsfläche ausgerichtete optische Achse aufweist,
wobei das Positionierungsgehäuse und das Erfassungsgehäuse eine gemeinsame Wand (114) aufweisen,
wobei die gemeinsame Wand eine Zieloberfläche (120) aufweist, die angeordnet ist, so dass die optische Achse der Erfassungsvorrichtung die Oberfläche schneidet, wobei die Zieloberfläche in Form einer Öffnung, die in das Positionierungsgehäuse und das Erfassungsgehäuse mündet, oder aus einem starren und transparenten Material ausgebildet ist,
- optisches Erkennen von Zeichen durch eine Recheneinheit (146) aus einem durch die Bilderfassungsvorrichtung erfassten Bild,
- Erzeugen von Ausgabedaten durch die Recheneinheit an eine Ausgabeeinrichtung.

## Claims

1. Device (100) for recognizing information data (126) present on a surface of an object (124) such as a vial, a syringe or an ampule, with a view to the secure production of medicinal preparations, comprising:
- a so-called positioning enclosure (118) which is arranged to receive the object and has a so-called reference surface (128) of a predetermined color,
- a so-called capturing enclosure (132) in which an image capturing device (136) is arranged, the image capturing device having an optical axis which is oriented in the direction of the reference surface,
wherein the positioning enclosure and the capturing enclosure have a common wall (114), said common wall having a target surface (120) which is arranged such that the optical axis of said capturing device intersects said target surface,
said target surface being made in the form of an opening which leads into the positioning enclosure and the capturing enclosure, or being made from a rigid and transparent material,
- a computation unit (146) which is configured to perform optical character recognition from an image captured by the image capturing device,
said computation unit being further configured to generate output data intended for an output peripheral.

2. Device according to the preceding claim, wherein the information data are carried by a label deposited on the surface of the object.

3. Device according to claim 1, wherein the information data are carried directly on the surface of the object.

4. Device according to any of the preceding claims, wherein the information data are represented in the form of characters and/or digits.

5. Device according to any of the preceding claims, wherein the information data are encoded as two-dimensional barcodes.

6. Device according to any of the preceding claims, wherein the output peripheral is a loudspeaker.

7. Device according to any of the preceding claims, further comprising a display device which is configured to display an image generated by the computation unit.

8. Device according to the preceding claim, wherein the image generated by the computation unit is produced from an image captured by the image capturing device and/or from output data.

9. Device according to any of the preceding claims, further comprising a device (144) for illuminating a region arranged in the capturing enclosure, on the optical axis, between the capturing device and the common wall.

10. Device according to the preceding claim, wherein the illumination device is an LED light panel.

11. Device according to any of the preceding claims, further comprising an internal electric battery.

12. Device according to any of the preceding claims, further provided with a wheeled structure, for example casters.

13. Method for recognizing information data present on a surface (126) of an object (124) such as a vial, a syringe or an ampule, with a view to the secure production of medicinal preparations, comprising:
- positioning the object in a so-called positioning enclosure (118), the positioning enclosure having a so-called reference surface (128) of a predetermined color,
- a capturing process in a so-called capturing enclosure (132) by an image capturing device (136), the image capturing device having an optical axis which is oriented in the direction of the reference surface,
wherein the positioning enclosure and the capturing enclosure have a common wall (114), said common wall having a target surface (120) which is arranged such that the optical axis of said capturing device intersects said surface, said target surface being made in the form of an opening which leads into the positioning enclosure and the capturing enclosure, or being made from a rigid and transparent material,
- optical character recognition by a computation unit (146) from an image captured by the image capturing device,
- generation of output data by the computation unit, which data are intended for an output peripheral.
